# EUROPEAN PATENT APPLICATION

(11) **EP 3 761 020 A1**
(43) Date of publication of application: **06.01.2021**
(21) Application number: 19184229.3
(22) Date of filing: 03.07.2019
(51) Int. Cl.: G01N 27/72, G01N 21/21, G01N 21/86, G01N 33/2045, G01R 33/032

(54) **NON-DESTRUCTIVE ANALYSIS OF ELECTRICAL STEEL USING A TRAINED ALGORITHM FOR MATERIAL PROPERTY ESTIMATION**

(71) Applicant: ABB Power Grids Switzerland AG, 5400 Baden (CH)
(72) Inventor: RUSSBERG, Gunnar, 724 62 Västerås (SE); WASS, Torbjörn, 723 55 Västerås (SE)
(74) Representative: Valea AB

(57) **Abstract**

The present disclosure relates to a method of analysing a grain oriented electrical steel (11). The method comprises, by means of a magnetic domain imaging unit (2), obtaining an image of the steel. The method also comprises, by means of a trained algorithm (5), estimating at least one property of the steel based on the obtained magnetic domain image.

## Description

### TECHNICAL FIELD

The present disclosure relates to a method of analysing a grain oriented electrical steel.

### BACKGROUND

Power and distribution transformers, shunt reactors, and electrical machines and generators, typically contain laminated cores of grain-oriented (GO) or non-oriented (NGO) electrical steels.

The electrical steels are classified by their composition and physical properties in different grades, separating materials of different magnetization behaviour, iron losses, magnetostriction, etc. For a specified grade one can associate nominal characteristics, such as BH-magnetization curves (where B is the magnetic flux density and H is the magnetic field strength) at given angles to the rolling direction, e.g. losses for different B, etc., by measuring a large number of samples and averaging the data. Such data are normally provided by the manufacturers. Statistical variations away from the nominal characteristics may or may not be available. In case no statistical information is available, or if one suspect, e.g., significant changes in quality over time, a transformer manufacturer can choose to regularly measure the characteristics of incoming material.

The local flux and loss distributions of most electrical steels are inhomogeneous on length scales less than a few centimetres and they are also strongly anisotropic. This is evident when viewing the magnetic domain pattern of the steel surface using, e.g., magneto-optical sensors. Thus there are expectedly large local variations in material performance and losses that smear out only when considering a large enough sample size of say a few decimetres or more in width.

The variance in actual physical characteristics within each steel grade or batch is thus not insignificant and, in the factory, other production steps like cutting, drilling and assembly are taking place which introduce stresses and irreversible changes in the material. All these factors influence the performance of the final transformer or machine core in an unpredictable way.

Growing demand for higher material quality and manufacturing quality requires new direct testing and measurement methods. Current methods to characterize the electrical steel material in the factory are destructive because samples are cut out from the steel roll in order to perform single-sheet measurements or measuring using an Epstein-frame. The process is relatively slow and expensive, since it involves several, often manual, preparation and measuring steps. It allows testing only a tiny fraction of the incoming material, cannot test the material being actually used, and it interferes with the flow of the manufacturing process. There is also a risk that the samples are not fully representative, since the mechanical handling slightly modifies their properties.

Brockhaus™ uses determination of magnetic flux density of a homogeneous magnetic field to calibrate with a very high accuracy magnetic properties like flux density and field strength.

### SUMMARY

It is an objective of the present invention to provide a solution for quick and non-destructive prediction of the material properties, in particular the magnetization and loss properties, of electrical steels e.g. for transformer cores.

According to an aspect of the present invention, there is provided a method of analysing a grain oriented electrical steel. The method comprises, by means of a magnetic domain imaging unit, obtaining an image of the steel. The method also comprises, by means of a trained algorithm, estimating at least one property of the steel based on the obtained magnetic domain image.

According to another aspect of the present invention, there is provided a system for analysing a grain oriented electrical steel. The system comprises a magnetic domain imaging unit configured to obtain an image of a grain oriented electrical steel. The system also comprises a digital data processing unit comprising a trained algorithm for estimating at least one property of the steel based on the obtained magnetic domain image.

Embodiments of the invention provide a passive and non-invasive method for magnetic properties analysis of the steel, without the need for e.g. cutting out samples of steel plate for analysis or for magnetizing the steel prior to analysis. It is relatively fast and can be performed in real time. Magnetic domain imaging may be used for visualizing the magnetic domains and the grains of the steel, but a trained algorithm as in accordance with the present invention is required for estimating the magnetic properties based on the image.

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:
Fig 1 is a schematic diagram of a system for analysing grain oriented electrical steels, in accordance with an embodiment of the present invention.
Fig 2 is a schematic flow chart of an embodiment of a method of the present invention.

### DETAILED DESCRIPTION

Embodiments will now be described more fully hereinafter with reference to the accompanying drawings, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description.

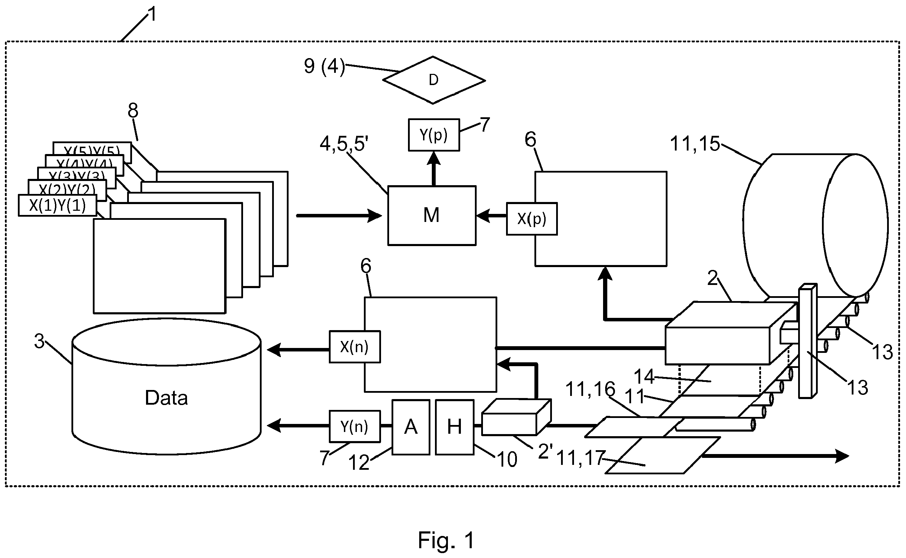

Figure 1 illustrates a system 1 for analysing a grain oriented electrical steel 11, typically in the form of a plate or stripe. The steel may be rolled onto a roll 15. A magnetic domain imaging unit 2, typically comprising a magneto-optical sensor, is arranged to obtain an image of the steel, e.g. as it is being rolled off the roll 15 but before it is being cut to parts 17 e.g. for forming a transformer core. Means 13 may be used for positioning the steel 11 and the imaging unit 2 in relation to each other, e.g. in the form of roll conveyor for the steel and a stand for the imaging unit as in figure 1.

Alternatively, quality mapping (e.g. including magnetic domain imaging) of already cut parts may be done later in the process, including during core assembly, or earlier in the process. For instance, it may be possible to unroll-reroll and map a plate batch after delivery to a customer. In general, the quality mapping may be executed at any stage from the final steel manufacturing step at a steel plant (i.e., before shipping) up to final core assembly in a transformer factory or even later, e.g., to get statistical data when disassembling old transformers, in failure investigations.

The imaging unit 2 is used to obtain a magnetic domain image 6 which is input to a digital data processing unit 4 comprising a trained algorithm 5 of the model M for estimating magnetic properties of the imaged steel. The algorithm may e.g. map data of the image 6 to material property characteristics 7, allowing a means 9 for automated decision making to estimate a magnetic property of the imaged steel. The means 9 for automated decision making may typically be comprised in the digital data processing unit 4, but may in some embodiments be arranged externally of the digital data processing unit 4 in a controller D. The digital data processing unit 4 typically comprises processing circuitry and a data storage storing instructions which, when executed by the processing circuitry, realizes the trained algorithm M.

The algorithm M may be pre-trained using image data 8 stored in a digital data storage unit 3 and/or in parallel with the estimating, e.g. by cutting out a sample 16 of the steel 11 for traditional analysis.

Such traditional analysis may include subjecting the sample 16 to a magnetic field provided by a means 10 to apply a magnetic field, after which the magnetized sample is imaged by means of a magnetic domain imaging unit 2', e.g. a second magnetic domain imaging unit which is different from the magnetic domain imaging unit 2. Alternatively, the image may be acquired already by the magnetic domain imaging unit 2 and the second magnetic domain imaging unit 2' may be superfluous, e.g. provided that the image can be precisely matched to the cut sample. A material property analyser 12 can then be used to obtain property characteristics 7 which are stored in the data storage 3 concurrently with image data 6.

Thus, an automated electrical steel quality recognition/prediction system 1 as exemplified in Figure 1 comprises a magnetic domain imaging unit 2 and a digital data processing unit 4, but may also comprise any of:
- A digital data storage unit 3.
- An adaptive mathematical model M 5 (i.e. the trained algorithm) which maps image 6 data X={Xi}, i=i,2,...,Nx obtained by the magnetic domain imaging unit 2 to material property characteristics Y={Yj}, j=i,2,...,Ny (7) determining the quality Q(Y).
- An adaptive mathematical model M 5' where M is, or involves, a machine learning algorithm with procedures for training, testing, and prediction.
- A data set {X(n),Y(n)}, n=1,2,... (8) obtained from magnetic domain imaging and material quality measurements, and used to optimize (train) the model M.
- Means 9 and procedure for automated decision making (analysis, logic, selection, control).
- Means 10 for magnetizing the test object 16.
- Means 12 for analysis (material property characterization) of the test object 16 and acquisition of the data.
- Means 13 for positioning the sensor imaging area 14 relative to the steel 11 to be imaged.
- An adaptive mathematical model M which involves an intermediate step (layer) with a state vector Z={Zi}, i=1,2,...,Nz having greatly reduced dimensionality Nz < < Nx and involving primarily physics based material characteristics which are not contained in material property characteristics Y.
- A mathematical model P predicting the performance of the final laminated core with input based on the predicted material nominal quality, on the laminations average quality, or on the individual lamination qualities.
- A wireless communication unit.

By quality is here meant a set of measurable or pre-defined material characteristics Y={Yj}, or a function thereof, Q(Y), that serves as differentiator for decisions and control of the manufacturing process. The quality is preferably quantified in terms of (i) a material grade, either the official grade of the manufacturer or a user defined sub-grade for finer classification, but could also be some representation of, e.g., (ii) the losses at different B, (iii) the magnetization behaviour, e.g., BH-curves, or (iv) the magnetostriction.

By machine learning is here meant any supervised or unsupervised learning method, or combination thereof, that is able to establish a reliable (e.g. at least 90% or at least 99%) coupling between input and output data. It may involve traditional data fitting and regression methods as well as modern Artificial Intelligence (AI) methods such as image/pattern recognition, dimension reduction, classification, clustering, anomaly detection, artificial neural networks, deep learning, image segmentation etc.

By imaging area is meant the surface area 14 of a steel sheet that is covered and/or depicted by the domain imaging unit 2 at a given time instant. If the image acquisition is made continuously, a quasi-iD (down to 1-pixel wide) scan line imaging may be performed, and the imaging area could be a suitably chosen number of such scan lines to define an area for image analysis.

By test object is here meant part of or all of an electrical steel sheet, which may be all material in a full roll 15 of steel (>100 metres), a small cut-out sample 16, or a ready-cut part 17 to be subsequently assembled in the core.

By decision making is understood that some action may be taken in response to the predicted quality of a test object, e.g., a sheet of core steel may be scrapped if it falls outside any classification or an anomaly is detected, or it may be selected for a specific purpose, e.g., assigned for assembly in a core with low (or high) loss evaluation if its predicted losses are sufficiently higher (or lower) than the nominal value.

A preferred magnetic domain imaging unit 2, or magnetic domain viewer, would comprise a magneto-optical sensor and, optionally, means for optical filtering and magnification. The magneto-optical principle is based on the Faraday effect which describes the rotation of the polarization plane of linearly polarized light when passing through a transparent medium. A direct, real-time visualization of a quasi-static magnetic field over the entire sensor surface is achieved. Commercial sensors can be customized in various geometries and sizes up to at least 75×75 mm for use in specific applications and they typically enable visualization of the vertical magnetic field from a flat surface with down to 1 µm spatial resolution and 1 µs time resolution. Other common magneto-optical methods utilize the magneto-optical Kerr effect on reflected polarized light and do not require a transparent sensor medium. Non-optical domain imaging techniques also exist but they may be less well suited for embodiments of the proposed invention: These include magnetic powder (Bitter colloids, ferrofluids, etc.) methods, transmission electron microscopy (TEM), reflection/scattering scanning electron microscopy (SEM), magnetic force microscopy (MFM), and X-ray and neutron methods.

The training data set outputs Y(n) (also called labels) may need to be determined from conventional destructive sampling + measurements unless a non-destructive conventional method is available, such as according to Brockhaus™ (mentioned above). After the initial training needed to obtain an acceptable first prediction reliability, prediction and continued training may run in parallel. The training data can be acquired by taking occasional samples 16 and analysing them at the actual steel handling process, or the acquisition and analysis 12 can be done separate from the site of quality control, e.g., in a dedicated laboratory with state-of-the-art material characterization equipment. The analysis typically requires means 10 for magnetizing the test objects 16 and for measuring magnetization curves and losses.

The positioning of the imaging area 14 may be in relative sense. The sensor unit 2 could be moving over a fixed test object 11, the test object could be passing a fixed sensor unit, small test objects 16 could be placed into a sample holder, or the sensor unit and test object could be both moving, step-wise or continuously. In case the sensor unit 2 does not require contact or near-contact with the test object but rather supports scanning at a distance the need for positioning actuators is reduced.

As intermediate physics based material characteristics, e.g. features when using AI or machine learning terminology, Z obtainable from the image data X one may consider averages and statistical variations of micro-structural characteristics like grain size, domain size, and domain orientation, as well as the density of laser scribing, etc. The final mapping from X to Z to Y then requires substantially less resources due to a massive reduction in dimensionality (Nx ∼10⁶→Nz ∼10² or less).

Embodiments of the present invention provides any of the following advantages:
- Offers quick, non-destructive electrical steel material characterization, classification, or quality control.
- Reduces labour and cost for material testing.
- Enables continuous real time material and core sheet testing, as well as occasional or frequent sample testing.
- Preferably integrated in the cutting and/or core assembly process.
- Reduces variations in magnetic core quality and performance.
- Reduces costs by reducing design margins.

A possible embodiment of the system 1 is as in Figure 1 in a transformer steel cutting process. A magnetic domain imaging unit 2 scans the steel 11 on-the-fly and produces image data 6. In an example embodiment, samples 16 are occasionally cut out for material characterization, or labelling, by analyser A, which associates a label Y(n) to the n^{th} sample (i.e., to image data X(n)). The labelled data is added to the data set being used to train the machine learning model M. The remaining and major part of the steel 11 is cut into parts (17) for utilization in core production. Each part p is associated with some input data X(p) for which a label Y(p) can be predicted by M. Based on Y(p) the controller (decision making unit) D decides how to treat the part in the remaining production steps, or Y(p) may be stored for post-manufacturing quality assessments and statistical analyses. The analyser A typically requires means 10 to apply a magnetic field H. A second domain imaging unit 2' may then be utilized for imaging material samples exposed to the field. The means 10, 12 and 2' are not necessarily physically located at the cutting site and the sample acquisition and analysis can be done completely independently of the cutting and manufacturing processes. In an alternative embodiment, the means 10 for applying a magnetic field and the material property analyser 12 are arranged together with the (first) magnetic domain imaging unit 2 and/or with the second magnetic domain imaging unit 2' in such a way as to analyse, non-destructively, parts of the utilized production steel instead of cutting out samples.

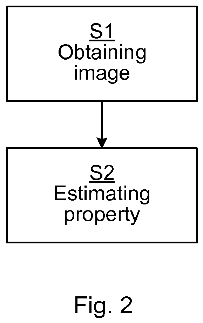

Figure 2 illustrates a method of the present invention. The method is for analysing a grain oriented electrical steel 11. The method comprises, by means of a magnetic domain imaging unit 2, obtaining S1 an image of the steel. The method also comprises, by means of a trained algorithm 5, estimating S2 at least one property of the steel based on the obtained S1 magnetic domain image.

In some embodiments of the present invention, the image is obtained without previous magnetisation of the steel.

In some embodiments of the present invention, the image is obtained on-the-fly, e.g. during cutting or core assembly of the steel.

In some embodiments of the present invention, the property is estimated in real time, e.g. during cutting or core assembly of the steel.

In some embodiments of the present invention, the steel is in form of a rolled steel plate passing the magnetic domain imaging unit, e.g. during cutting or core assembly of the steel.

In some embodiments of the present invention, the algorithm is an AI algorithm. A goal of Artificial Intelligence (AI) may be to create the algorithm that can identify various problems and then devise its own algorithms to solve these problems. AI could be said to have an ingredient of learning from experience and adapt its output to new input data. Embodiments of the present invention may not necessarily have to adapt to new data once the basic training has been performed. Thus, the original trained model (algorithm) may be enough for operation in accordance with some embodiments of the present invention.

In some embodiments of the present invention, the at least one property comprises any of a magnetization property, e.g. magnetostriction, and a magnetic loss property.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A method of analysing a grain oriented electrical steel (11), the method comprising:
by means of a magnetic domain imaging unit (2), obtaining (Si) an image of the steel; and
by means of a trained algorithm (5), estimating (S2) at least one property of the steel based on the obtained (Si) magnetic domain image.

2. The method of claim 1, wherein the image is obtained without previous magnetisation of the steel.

3. The method of any preceding claim, wherein the image is obtained on-the-fly, e.g. during cutting or core assembly of the steel.

4. The method of any preceding claim, wherein the property is estimated in real time, e.g. during cutting or core assembly of the steel.

5. The method of any preceding claim, wherein the steel is in form of a rolled steel plate passing the magnetic domain imaging unit, e.g. during cutting or core assembly of the steel.

6. The method of any preceding claim, wherein the algorithm is an AI algorithm.

7. The method of any preceding claim, wherein the at least one property comprises any of a magnetization property, e.g. magnetostriction, and a magnetic loss property.

8. A system (1) for analysing a grain oriented electrical steel, the system comprising:
a magnetic domain imaging unit (2) configured to obtain an image of a grain oriented electrical steel; and
a digital data processing unit (4) comprising a trained algorithm (M) for estimating at least one property of the steel based on the obtained magnetic domain image.
